# EUROPÄISCHE PATENTANMELDUNG

(11) **EP 2 703 002 A1**
(43) Veröffentlichungstag der Anmeldung: **05.03.2014**
(21) Anmeldenummer: 12006150.2
(22) Anmeldetag: 30.08.2012
(51) Int. Cl.: A61K 36/18, A61P 25/24

(54) **Pflanzliche Extrakte aus der Nachtkerze (Oenothera biennis) zur Behandlung von Störungen des zentralen Nervensystems**

(71) Anmelder: Mewicon med. wiss. Beratung GmbH, 4164 Schwarzenberg (AT)
(72) Erfinder: Dimpfel, Wilfried, 35578 Wetzlar (DE)

(57) **Zusammenfassung**

Die vorliegende Erfindung betrifft Oenothera Extrakt, Oenothera Extraktderivate und/oder dessen Inhaltsstoffe als Modulator des glutamatergen Systems, insbesondere als Antagonist von Glutamatrezeptoren. Die vorliegende Erfindung betrifft auch Oenothera Extrakt, Oenothera Extraktderivate und/oder dessen Inhaltsstoffe zur Behandlung von Störungen des zentralen Nervensystems, insbesondere bei Morbus Parkinson, Demenzen verschiedener Genese, sowie beim Erschöpfungs-Syndrom (adaptogene Wirkung). Die vorliegende betrifft auch ein Arzneimittel zur Behandlung von Störungen des zentralen Nervensystems, insbesondere bei Morbus Parkinson, Demenzen verschiedener Genese, sowie beim Stress-induziertem Erschöpfungs-Syndrom enthaltend Oenothera Extrakt, dessen Derivate und/oder dessen Inhaltsstoffe und ggf. übliche Hilfs- und Zusatzstoffe.

## Beschreibung

Die vorliegende Erfindung betrifft pflanzliche Extrakte, die aus der Nachtkerze (Oenothera) hergestellt werden und die Aktivität des glutamatergen Neurotransmittersystems beeinflussen. Die vorliegende Erfindung betrifft auch Oenothera Extrakte zur Behandlung von Störungen des zentralen Nervensystems, insbesondere bei Morbus Parkinson, Demenzen verschiedener Genese, sowie Depressionen und Erschöpfung. Die vorliegende Erfindung betrifft auch ein Arzneimittel, welches eine sogenannte adaptogene Wirkung enfaltet. Adaptogene sind eine Wirkstoffklasse, die auf Grund ihrer Wirkung bei Stress-induzierter Übermüdung und Erschöpfung eine bessere Anpassung des Körpers erlaubt. Der Körper bzw. sein Immunsystem soll besser an den Stress adaptiert (angepasst) werden. Bekannte Vertreter dieser Wirkstoffklasse sind z.B. Extrakte von Rosenwurz (Rhodiola rosea) und Ginseng sowie Schisandra. Adaptogene sollen völlig ungiftig sein und breite positive Wirkung haben (Wikipedia).

Die Gemeine Nachtkerze (Z.B. Oenothera paradoxa) gehört zur Gattung der Nachtkerzengewächse (Onagraceae). Das aus ihren Samen hergestellte Nachtkerzenöl hat entzündunghemmende Eigenschaften und wird zur Behandlung der Neurodermitis verwendet. Ein direkte Wirkung auf das Nervensystem wurde noch nicht beschrieben.

Extrakte von Oenothera paradoxa enthalten einen hohen Anteil von Polyphenolen (bis 50%).

Die derzeit üblichen Arzneimittel zur Behandlung von Störungen des zentralen Nervensystems, insbesondere von Demenzen, Morbus Parkinson, sowie Depressionen haben ein breites Nebenwirkungsspektrum. Insbesondere die Behandlung der Depression wird mit Paroxetin durchgeführt, obwohl dieses Medikament erhebliche Nebenwirkungen besitzt. Es besteht daher ein großer Bedarf nach einem verbesserten Arzneimittel mit einer guten therapeutischen Wirksamkeit bei möglichst geringer Nebenwirkungsrate. Diese geringe Nebenwirkungsrate ist bei Verabreichung einer pflanzlichen Naturstoffmischung, insbesondere mit adaptogener Wirkung wie bei Rhodiola rosea, eher zu erwarten.

Überraschend wurde nunmehr gefunden, daß Oenothera Extrakte eine biologische oder pharmazeutische Wirkung in Bezug auf Himfunktionen aufweist und zwar im Sinne einer schützenden adaptogenen Substanz. Die Anwendung der völlig "bias"-freien Untersuchung im Tele-Stereo-EEG der Ratte erbrachte im Rahmen von Versuchen mit anderer Zielsetzung den Hinweis auf das Vorhandensein einer derartigen adapatogenen Wirkung im Bereich gestörter Hirnfunktionen. Die überraschend gefundene biologische oder pharmazeutische Wirkung bzw. Aktiviät wurde in vivo an der Ratte und in vitro dokumentiert.

Es wird angenommen, dass die vorgenannte Aktivität auf einer Wirkung von Oenothera Extrakten auf das glutamaterge System beruht.

Die Erfindung betrifft daher Oenothera Extrakte zur Behandlung von Störungen des zentralen Nervensystems, insbesondere bei degenerativen Erkrankungen, wie Demenzen, Morbus Parkinson und Stress-induziertem Erschöpfungs-Syndrom sowie "Burnout".

Im Rahmen der vorliegenden Erfindung sollen, sofern nicht anders angegeben, von dem Begriff "Oenothera Extrakte" alle Erscheinungsformen, d.h. jede Art der Herstellung derartiger Extakte aus der Nachtkerze sowie deren Inhaltsstoffe umfaßt sein. Besonders bevorzugt wird Oenothera Extrakt als Trockenextrakt eingesetzt.

Unter zentralnervösen Störungen sollen vorliegend insbesondere Konzentrationsstörungen wie sie im Rahmen einer Depression beschrieben werden, Gedächtnisstörungen wie sie im Rahmen des Vorliegens einer Alzheimer'schen Krankheit vorliegen, Stimmungsschwankungen wie sie nach starker Erschöpfung auftreten, Orientierungsstörungen wie sie im Rahmen einer Demenz vorkommen, Störungen der motorischen Kontrolle, wie sie bei Vorliegen der Parkinson'schen Krankheit beschrieben werden.

Unter üblichen Dosierungen werden vorliegend Dosen im Bereich von ca. 200 mg bis ca. 800 mg am Tag verstanden. Niedrige Dosierungen betreffen Dosen kleiner ca. 200 mg am Tag bis ca. 50 mg am Tag.

In in vitro Untersuchungen konnte eine Wirkung von Oenothera Extrakt auf die Aktivität der glutamatergen Transmission im Hippokampus nachgewiesen werden.

Die vorliegende Erfindung betrifft daher auch die Eigenschaften von Oenothera Extrakt, Extraktderivaten und/oder deren Inahltsstoffen als Modulator des glutamatergen Systems, bevorzugt als Antagonist. Entsprechend ist ein weiterer Gegenstand der vorliegenden Erfindung Oenothera Extrakt, Oenothera Extraktderivate und/oder dessen Inhaltsstoffe zur Behandlung von Zuständen, die durch eine Überaktivität des glutamatergen Systems bedingt sind. Derartige Zustände sind beispielsweise Störungen und Beeinflussungen der Modulation synaptischer Übertragung und der Induktion synaptischer Plasizität, wie (Langzeit-Potenzierung und (Langzeit-)Depression der synaptischen Signalübertragung.

Die vorliegende Erfindung betrifft auch die Verwendung von Oenothera Extakten zur Behandlung von Störungen des zentralen Nervensystems, insbesondere bei degenerativen Erkrankungen, wie Demenzen, Morbus Parkinson und dem Erschöpfungs-Syndrom. Die Erfindung betrifft auch die Verwendung von Oenothera Extakaten als Mittel zur Behandlung von Störungen des zentralen Nervensystems, insbesondere bei degenerativen Erkrankungen, wie Demenzen, Morbus Parkinson und dem Stress-induzierten Erschöpfungs-Syndrom, sowie deren Verwendung zur Herstellung entsprechender Mittel. In diesem Zusammenhang ist auch die Herrichtung von Oenothera Extrakten als ein entsprechendes Mittel enthalten.

Zusätzlicher Gegenstand der Erfindung ist ein Verfahren zur Behandlung von Störungen des zentralen Nervensystems, insbesondere bei degenerativen Erkrankungen, wie Demenzen, Morbus Parkinson und dem Stress-induzierten Erschöpfungs-Syndrom umfassend die Gabe von Oenothera Extrakten der eines erfindungsgemäßen Mittels an einen Patienten, der eine solche Behandlung benötigt in einer therapeutisch wirksamen Menge.

Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind. Soweit nichts anderes angegeben ist oder sich aus dem Zusammenhang zwingend anders ergibt, beziehen sich Prozentangaben auf das Gewicht, im Zweifel auf das Gesamtgewicht der Mischung.

Die erfindungsgemäßen Extrakte aus Oenothera als Wirkstoff enthaltenden (Arznei)Mittel werden bevorzugt oral verabreicht. Es ist jedoch auch eine Verabreichung auf anderem Wege, z.B. peroral, topisch, parenteral, intravenös, intramuskulär, subkutan, nasal, inhalativ oder transdermal möglich.

Zur Verabreichung kann das erfindungsgemäße Mittel z.B. in Form von Tabletten, Kapseln, Pillen, Dragees, Granulaten, Suppositorien, Pellets, Lösungen, Dispersionen formuliert werden, wobei optional pharmazeutisch annehmbare Hilfs- und Trägerstoffe enthalten sind.

Liegt das erfindungsgemäße Mittel in oraler Darreichungsform z.B. als eine Lösung vor, so enthält diese typischerweise 200mg /ml, bevorzugt 100 mg /ml des Oenothera Extraktes. Andere Gehalte sind möglich und können vom Fachmann mittels einfacher Versuche, ggf. unter Berücksichtigung des Alters, Gewichts, Geschlechts und weiterer Parameter eines konkreten Patienten ermittelt werden.

Die erfindungsgemäßen Mittel werden normalerweise gemäss herkömmlichen Methoden hergestellt und in einer pharmazeutisch geeigneten Form verabreicht.

Zum Beispiel können die festen oralen Formen zusammen mit dem Wirkstoff Streckstoffe, z.B. Lactose, Dextrose, Saccharose, Cellulose, Maisstärke oder Kartoffelstärke; Gleitmittel, z.B. Silikat, Talk, Stearinsäure, Magnesium- oder Calciumstearat und/oder Polyethylenglykole; Bindemittel, z.B. Stärken, Gummi arabicum, Gelatine, Methylcellulose, Carboxymethylcellulose oder Polyvinylpyrrolidon: Quell- und Sprengmittel, z.B. Stärke, Alginsäure, Alginate oder Natriumstärkeglykolate, aufschäumende Mischungen; Farbstoffe; Süßungsmittel; Benetzungsmittel, wie Lecithin, Polysorbate, Laurylsulfate; und im allgemeinen nicht-toxische und pharmakologisch inaktive Substanzen, die in pharmazeutischen Formulierungen verwendet werden, enthalten.

Die pharmazeutischen Zubereitungen können in bekannter Weise hergestellt werden, z.B. mittels Mischen, Granulieren, Tablettieren, Zuckerbeschichtungs- oder Überzugsbeschichtungsverfahren.

Flüssige Darreichungsformen zur oralen Verabreichung können z.B. Sirupe, Emulsionen und Suspensionen oder Dispersionen sein.

Ein Sirup kann als Träger z.B. Saccharose oder Saccharose mit Glycerin und/oder Mannitol und/oder Sorbitol enthalten.

Suspensionen und Emulsionen können als Träger z.B. ein natürliches Harz, Agar, Natriumalginat, Pectin, Methylcellulose, Carboxymethylcellulose oder Polyvinylalkohol enthalten.

Suspensionen oder Lösungen für intramuskuläre Injektionen können zusammen mit dem Wirkstoff einen pharmazeutisch annehmbaren Träger, z.B. steriles Wasser, Olivenöl, Ehyloleat, Glykole, z.B. Propylenglykol, und, erforderlichenfalls, eine geeignete Menge an Lidocain-Hydrochlorid enthalten.

Lösungen zur intravenösen Injektion oder Infusion können als Träger z.B. steriles Wasser enthalten oder sie können bevorzugt in Form von sterilen, wässrigen, isotonischen Salzlösungen vorliegen.

Suppositorien können zusammen mit dem Wirkstoff einen pharmazeutisch annehmbaren Träger, z.B. Kakaobutter, Polyethylenglykol, einen Polyoxyethylensorbitol-Fettsäureester oder Lecithin enthalten.

Zusammensetzungen für die topische Applikation, z.B. Cremes, Lotionen oder Pasten, können durch Mischen des Wirkstoffs mit einem herkömmlichen ölhaltigen oder emulgierenden Träger hergestellt werden.

Eine Dosierungseinheit des Mittels kann beispielsweise enthalten:
bei peroralen Arzneiformen:
   100 - 200 mg, bevorzugt 150 mg, besonders bevorzugt 120 bis 140 mg Extrakt pro Tagesdosis.
   Die Tagesdosis kann beispielsweise in 1 bis 3 Einzeldosen, vorzugsweise in zwei Einzeldosen, täglich verabreicht werden.

Eine Anpassung der konkreten Dosis in Abhängigkeit vom Alter, Geschlecht, Gewicht, Zustand, etc. des Patienten und des verwendeten Extraktes liegt im Können des Fachmanns und erfolgt auf dem Fachmann bekannte Weise.

Nachfolgend wird eine kurze Beschreibung der Abbildungen gegeben.

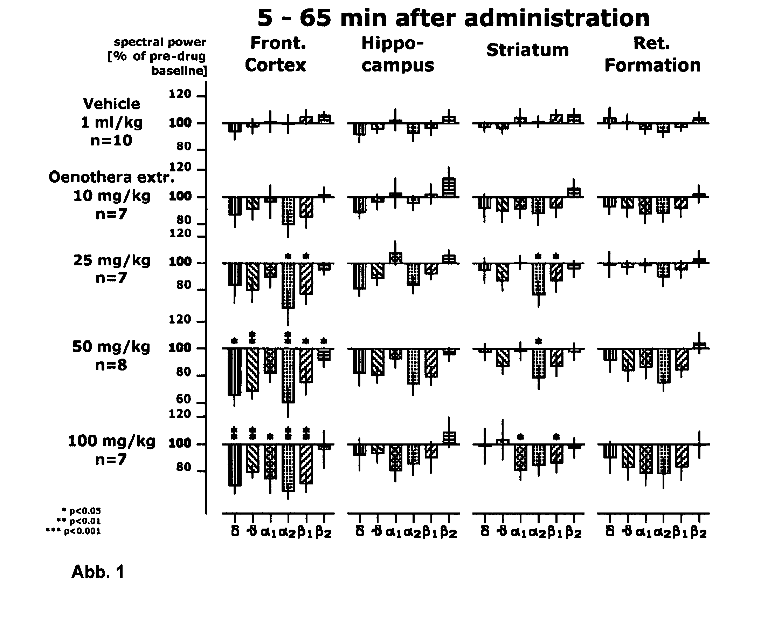

Abb. 1 zeigt die Dosis-abhängige Wirkung von Oenothera Extrakt auf EEG Frequenzen im Vergleich zur Kontrolle (Wasser als Vehikel). Mittelwerte von n=7-10 Tieren im jeweiligen Experiment. Auf der y-Achse ist die Dosis aufgetragen. Die x-Achse gibt die Frequenzbereiche nach der Fast Fourier Transformation der Daten an: delta (δ), theta (θ), alpha1 (α1), alpha2 (α2), beta1 (β1) und beta2 (β2). Mittelwerte von n=12 Tieren. Sterne kennzeichnen die statistische Signifikanz, berechnet nach Wilcoxon, Mann und Whitney, zweiseitig: *=p<0.05; **=p<0.01; ***=p<0.001.

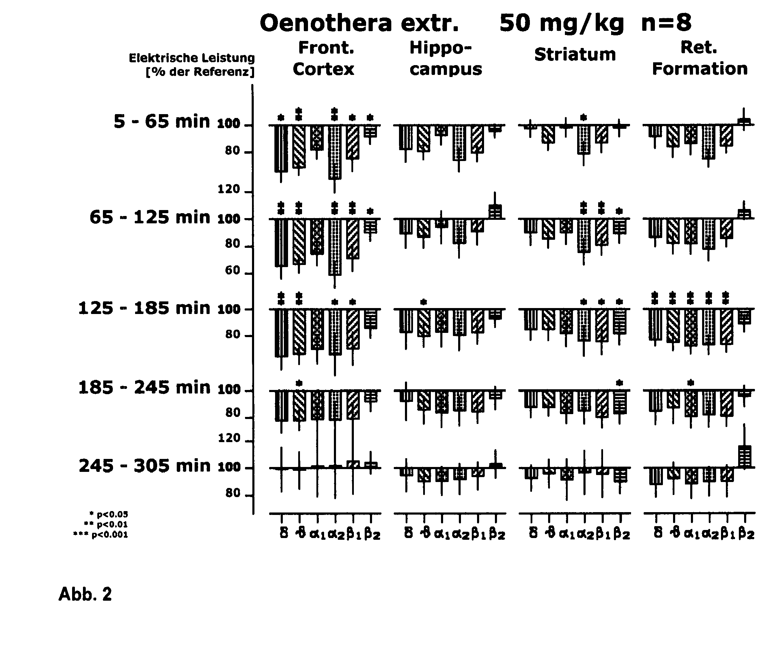

Abb. 2 zeigt eine Zeit-abhängige Wirkung von Oenothera Extrakt in vivo im Vergleich zur Applikation von Vehikel (Dosis: 50 mg/kg). Weitere Details s. Abb. 1.

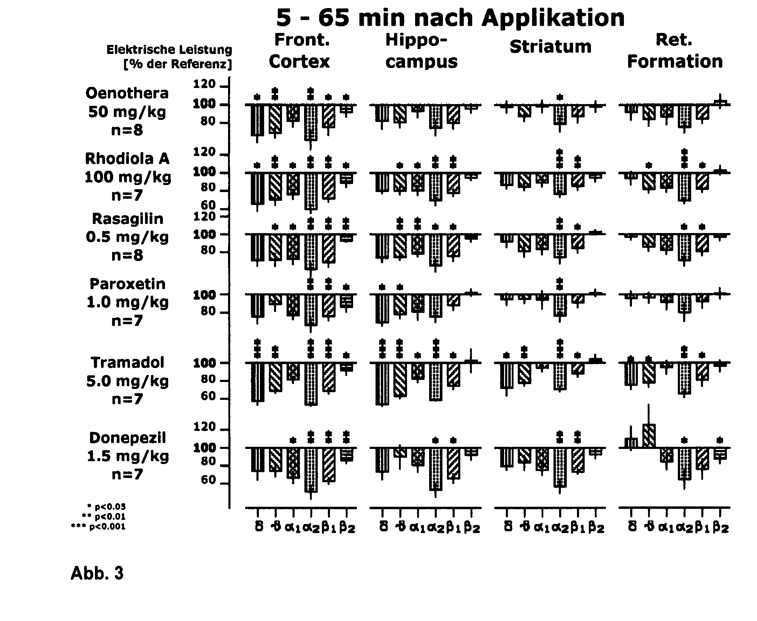

Abb. 3 zeigt die Dokumentation des Vergleiches der Wirkung von Oenothera Extrakt mit Referenzmedikamenten wie Rhodiola rosea (Adaptogen), Rasagilin (Mittel zur Behandlung des Morbus Parkinson), Donepezil (Mittel zur Behandlung von Demenzen), Paroxetin (Mittel gegen Depressionen) und Tramadol, einem Analgetikum.

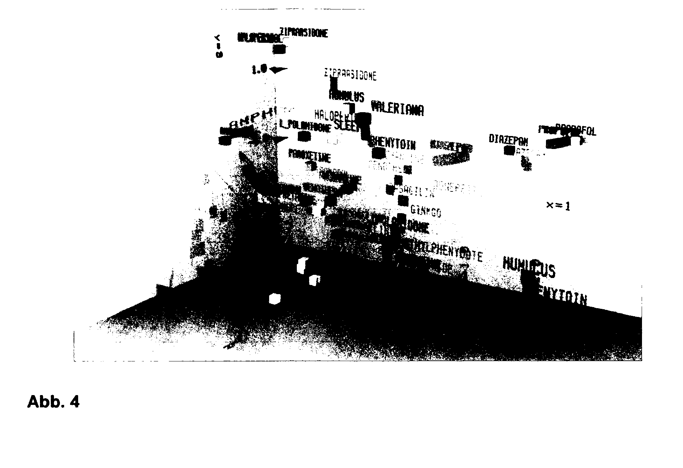

Abb. 4 zeigt die Wirkung von Oenothera Extrakt im Vergleich zu Referenzpharmaka mit bekannter klinischer Wirkung. Auf Grund der Position können große Ähnlichkeit zu Präparaten wie Rhodiola rosea, Rasagilin, Donepezil, Paroxetin und Tramadol anhand der Ergebnisse einer linearen Diskriminanzanalyse auf der Basis von vier Hirnregionen und 6 Frequenzbändern (24 Variablen) erkannt werden. Medikamente sind mit vollem Namen angegeben. Die Ergebnisse der ersten und zweiten Diskriminanzfunktion sind auf der x- und y-Achse dargestellt. Das Ergebnis der dritten Diskriminanzfunktion befindet sich auf der z-Achse. Die Ergebnisse der vierten bis sechsten Diskriminanzfunktionen sind anhand einer Farbmischung dargestellt, sind jedoch in dieser nicht farbigen Darstellung nicht sichtbar. Die sich ergebenden Farben (hier vorliegend in Graustufen dargestellt) entstehen aus einer additiven Farbmischung aus den Farben rot-grün-blau stellvertretend für die Ergebnisse der vierten, fünften und sechsten Diskriminanuzfunktion. Die Wirkung von Oenothera Extrakt ordnet sich in die Nachbarschaft von Tramadol (Analgetikum), Rasagilin (Medikament gegen Morbus Parkinson), Paroxetin (Mittel gegen Depressionen) und Donepezil (Medikament zur Behandlung des Morbus Alzheimer) zum Teil auch ähnliche Farbe, d.h. in dieser Darstellung als Nachbarn mit ähnlicher Graustufe).

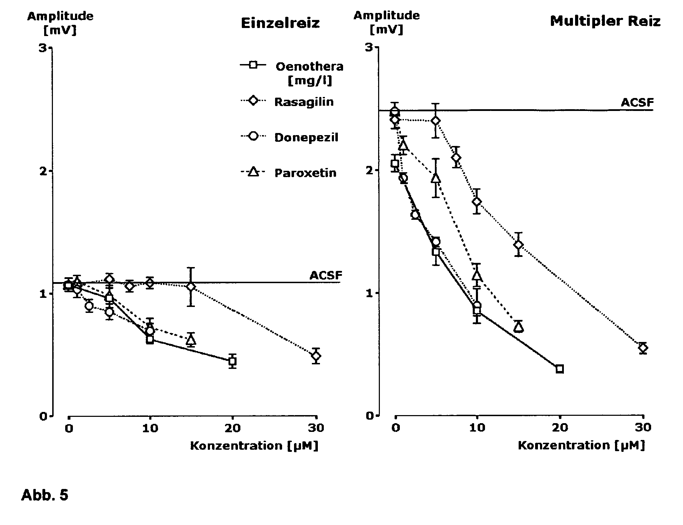

Abb. 5 zeigt die Wirkung von Oenothera Extrakt im Hippokampus-Schnittpräparat in vitro. Die Erfassung des Populationsspikes der Pyramidenzellen erfolgt nach elektrischer Reizung der Schaffer-Kollateralen in Form von Einzelimpulsen sowie in Gegenwart multipler Reize zur Darstellung der Langzeitpotenzierung. Statistische Signifikanzen sind in Form einer Tabelle dargestellt:

**Tabelle : Aufistung der statistischen Signifikanzen bezüglich der in Abb. 5 dargestellten Substanz- bzw. Präparatewirkungen. Man beachte, dass für die synthetischen Präparate dien Angabe in Molarität erfolgt, für den Pflanze.nextrakt aus Oenothera jedoch als mg/L.**

| **Dosis** | **Oenothera ([mg/l])** | | **Rasagilin** | | **Donepezil** | | **Paroxetin** | |
|---|---|---|---|---|---|---|---|---|
| **[µM]** | **SS** | **TBS** | **SS** | **TBS** | **SS** | **TBS** | **SS** | **TBS** |
| **1.0** | | | | | n.s. | p<0.01 | p<0.10 | p<0.10 |
| **2.5** | n.s. | n.s. | | | p<0.01 | p<0.01 | | |
| **5.0** | p<0.02 | p<0.01 | n.s | n.s | p<0.01 | p<0.01 | p<0.05 | p<0.05 |
| **7.5** | | | n.s. | p<0.10 | | | | |
| **10.0** | p<0.01 | p<0.01 | n.s. | p<0.01 | p<0.01 | p<0.01 | p<0.01 | p<0.01 |
| **15.0** | | | n.s. | p<0.01 | | | p<0.01 | p<0.01 |
| **20.0** | p<0.01 | p<0.01 | | | | | | |
| **30.0** | | | n.s | p<0.01 | | | | |

### Beispiel 1 (Tele-Stereo-EEG)

Die Veränderungen der EEG-Frequenzen wurde nach oraler Gabe von Wasser (Kontrolle/Vehikel) bzw. Verdünnungen des Oenothera Extraktes Wasser entsprechend 10 mg/kg, 25 mg/kg, 50 mg/kg und 100 mg/kg_Körpergewicht bestimmt.

Die Untersuchungen wurden analog der durch W. Dimpfel beschriebenen Methode (Dimpfel W Preclinical) data base of pharmaco-specific rat EEG fingerprints (Tele-Stereo-EEG). Eur J Med Res (2003) 8: 199-207; auf dieses Dokument wird vollinhaltlich Bezug genommen) folgendermaßen durchgeführt:

Zehn männlichen erwachsenen Fischer-344 Ratten (Tag-Nacht konvertiert) wurden im Alter von 6 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippokampus, Striatum und Formatio Reticularis abgeleiteten Feldpotentiale. Die Signale wurden auf einem Computer System (Software "EEG-Analyse", Betriebssystem OS Science, Laborrechner "LabTeam" der Firma MediSyst, Linden, DE) in Echtzeit einer Fast-FourierTransformation unterworfen und die Leistungsdichtespektren jeweils über 60 Minuten gemittelt. Die Unterteilung der Spektren in 6 verschiedene Frequenzbereiche erlaubte die Erfassung pharmako-spezifischer Veränderungen in Bezug auf die jeweils vor Applikation gemessenen Vorwerte innerhalb dieser Frequenzbänder.

Zum Applikationsprotokoll der Substanzen: die Substanzen wurden oral 45 Minuten nach Beginn der Messungen (Vorwert) appliziert. Fünf Minuten später wurden die Messungen wieder gestartet, mindestens über die nächsten 5 Stunden kontinuierlich analysiert und in 60-minütigen Perioden zusammengefasst. Der Extrakt wurde in einer Dosierung 10, 25, 50 und 100mg/kg appliziert. Die experimentelle Serie wurde mit der Applikation von Wasser (Kontrolle), die zu keiner auffälligen Änderung führte, begonnen (oberste Zeile in Abb. 1).

Der statistische Vergleich der Versuche zu den Ergebnissen, die nach Gabe des Vehikels gemessen wurde, erfolgte mit Hilfe des statistischen Tests nach Wilcoxon, Mann und Whitney auf der Basis der Veränderungen innerhalb der einzelnen Frequenzbänder in allen Hirnregionen als Variablen.

Die Verabreichung von Wasser führte kaum zu Veränderungen der elektrischen Leistung (µV²) im Vergleich zu den Vorphasenwerten (Abb. 1).

Die Verabreichung von Oenothera Extrakt führte zu einer dosisabhängigen Unterdrückung der spektralen Leistung in mehreren Frequenzbereichen. Besonders stark waren die alpha2 und beta1 Wellen betroffen. In höheren Dosierungen (50 und 100 mg/kg) kam es auch zu einer Unterdrückung der delta und theta Wellen. Eine Steigerung der Motilität der Tiere wurde nicht beobachtet. Der zeitabhängige Verlauf der Wirkung ist in Abb. 2 dargestellt. Die Wirkung konnte auch in der 4. Stunde nach oraler Gabe noch nachgewiesen werden.

Die Untersuchungen der Veränderungen der EEG-Frequenzen der Ratte nach Gabe von Oehothera Extrakt zeigen überraschenderweise im Vergleich zur Gabe von Vehikel (Trägersubstanz Wasser), dass das Präparat eine Wirkung im Gehirn besitzt, die bei der Behandlung von Beeinträchtigungen des Gehirns, insbesondere bei Morbus Parkinson, Demenzen verschiedener Genese, sowie beim Stress-induziertenErschöpfungs-Syndrom (adaptogene Wirkung) nützlich ist (Abb.3).

Die Abb. 3 zeigt die Veränderungen der EEG-Frequenzen nach Gabe des Oenothera Extaktes im Vergleich zu verschiedenen Medikamenten mit bekannter klinischer Indikation. Oenothera Extrakt zeigt überraschenderweise im Modell Tele-Stereo-EEG bei Ratten dosisabhängige Veränderungen der EEG-Frequenzen, wie sie nach Gabe klassischer Medikamente zur Behandlung Demenzen (Beispiel Donepezil), Morbus Parkinson (Beispiel Rasagilin), wie auch beim Erschöpfungs-Syndrom (Beispiel Rhodioloa rosea) gemessen werden. Die Veränderungen, wie sie nach Gabe von einer Dosis von Oenothera Extrakt (50 und 100 mg/kg) auftreten, sowie nach Gabe klassischer Medikamente zur Behandlung degenerativer Krankheiten mit Beeinträchtigung kognitiver Funktionen verwendet werden (Abb. 3) zeigen eine große Ähnlichkeit untereinander. Die in vivo Versuche an der Ratte zeigen somit für Oenothera Extrakt Ergebnisse, wie sie für Medikamente zur Behandlung von Demenzen, Morbus Parkinson sowie Erschöpfungs-Syndromen typisch sind (Abb. 3). Insbesondere fällt die nahezu identische Wirkung im Vergleich zu dem Adaptogen Rhodiola rosea auf. Adaptogene stellen eine eigene alternativ-medizinische Wirkstoffklasse dar, die nicht nur zur Behandlung von Erschöpfungs-Syndromen verwendet wird, sondern auch Stress und depressiven Verstimmungen entgegenwirkt.

Die beobachteten Frequenzänderungen wurden mittels einer Diskriminanzanalyse mit den Ergebnissen von Medikamenten zur Behandlung von degenerativen Erkrankungen des Gehirns wie auch mit Medikamenten für andere Indikationen verglichen. Arzneimittel, welche bereits im selben Modell unter identischen Bedingungen geprüft wurden, standen für Vergleichszwecke zur Verfügung (siehe nachfolgendeTabelle). Die Angaben dort beziehen sich jeweils auf eine Dosierung in mg/kg Körpergewicht.

**Tabelle: Auflistung der in der Diskriminanzanalyse verwendeten Referenzsubstanzen. Die unter Definition gelisteten Substanzen wurden für die Erstellung der Diskriminanzfunktionen verwendet. Die unter Analyse aufgelisteten Substanzen wurden unter Verwendung dieser Funktionen unter Verwendung derselben Projektionsparameter abgebildet.**

| **Substanzen Definition** | **Dosis [mg/kg]** | **Zeit** | **Substanzen Analyse** | **Dosisw [mg/kg]** | **Zeit** |
|---|---|---|---|---|---|
| Diazepam | 0.50 | 5 - 35 min | Caffein | 2.50 | 5 - 35 min |
| Memantin | 3.00 | 5 - 35 min | Paullinia | 15.0 | 5 - 65 min |
| L-Polamidon | 1.00 | 5 - 35 min | Ginkgo extr. | 100 | 5 - 65 min |
| Ziprasidon | 1.00 | 5 - 35 min | Oenothera | 50 | 5 - 65 min |
| Haloperidol | 0.50 | 5 - 35 min | Rhodiola A | 100 | 5 - 65 min |
| (+)Amphetamin | 0.40 | 5 - 65 min | Donepezil | 1.50 | 5 - 65 min |
| Paroxetin | 1.00 | 5 - 35 min | Rasagilin | 0.50 | 5 - 65 min |
| Ziprasidon | 1.00 | 5 - 35 min | | | |
| Propofol | 60.0 | 5 - 65 min | Valeriana 185 | 60 | 125 - 185 min |
| Dizocilpin | 0.25 | 5 - 35 min | Humulus | 50 | 125 - 185 min |
| Moclobemid | 5.00 | 5 - 35 min | | | |
| Phenytoin | 4.00 | 65 - 125 min | | | |
| Sleep | | 65 - 125 min | | | |
| Tramadol | 5.00 | 5 - 35 min | | | |

Das aufgefundene Frequenzmuster für Oenothera Extrakt korreliert in auffälliger Weise mit den Mustern, die für Medikamente, wie sie bereits für die Behandlung von Demenz (Donepezil), Morbus Parkinson (Rasagilin) sowie Erschöpfungs-Syndrom (Rhodiola rosea) üblicherweise verwendet werden, nicht jedoch mit Mustern, die bei Medikamenten für andere Indikationen auftreten (Abb. 3).

Die Diskriminanzanalyse hat gezeigt, dass sich der charakteristische elektrische Fingerprint, der für klassische Medikamente zur Behandlung von Demenz, Morbus Parkinson, sowie Erschöpfungs-Syndromen typisch ist, auch in den Mustern von Oenothera Extrakt in auffälliger Weise wiederfindet. Aus der Erkenntnis, dass Oenothera Extrakt die gleichen charakteristischen Veränderungen der EEG-Frequenzen hervorruft wie üblicherweise Arzneimittel, die zur Behandlung von Demenz, Morbus Parkinson, und Erschöpfungs-Syndromen eingesetzt werden, kann gefolgert werden, dass Oenothera Extrakt bei der Behandlung in denselben klinischen Indikationen wirksam ist. Dass Medikamente, die in der gleichen Indikation eingesetzt werden, auch gleichartige EEG Veränderungen hervorrufen, konnte von Dimpfel anhand von mehr als 40 Referenzsubstanzen für 8 verschiedene Indikationen gezeigt werden (Dimpfel W Preclinical data base of pharmaco-specific rat EEG fingerprints (Tele-Stereo-EEG). Eur J Med Res (2003) 8: 199-207).

### Beispiel 2 (Langzeitpotenzierung im Hippokampus-Schnittpräparat in vitro)

Wirkungen von Oenothera Extrakt auf die Erregbarkeit der Pyramidenzellen wurden im Hippokampus-Schnittpräparat der Ratte bestimmt. Die Analyse der Wirkungen erfolgte auf zwei Ebenen. Zunächst wurde anhand der Applikation von Einzelreizen die normale Erregbarkeit überprüft und anschließend nach einer sog. "Theta-burst-Stimulation" die Auswirkungen auf die Langzeitpotenzierung gemessen

Für die Durchführung dieser Studien wurden 6 erwachsene männliche CD-Ratten verwendet. Die Isolation des Hippokampus erfolgte an äthernarkotisierten und anschließend exsanguinierten Tieren. In Phosphatgepufferter Salzlösung (NaCl: 124 mM; KCI: 5 mM; CaCl: 2 mM; MgSO4: 2 mM; NaH2PO4: 1,25 mM; NaHCO3: 26 mM: Glucose: 10 mM; Kontroll-Lösung: Artifizielle Cerebral-Spinal-FLüsssigkeit (ACSF); alle Substanzen stammen von der Firma Roth, Karlsruhe, DE. Dann wurde der mittlere Teil des Hippokampus mit Hilfe eines Schnellklebers aufgeblockt und mit einem Vibratom in 400 µm dicke Scheiben geschnitten. Die Aufbewahrung dieser Schnitte erfolgte für mindestens eine Stunde vor Versuchsbeginn (siehe S.J. Schiff, G.G. Somjen "The effects of temperature on synaptic transmission in hippocampal tissue slices" Brain Research (1985), 345: 279-284) in einer mit Carbogen durchperlten Inkubationskammer.

Das Experiment selbst wurde in einer sog. "Base Unit" mit Haas Top" (Firma Medical Systems Corp., USA) bei 35 Grad Celsius durchgeführt. Der mit Hilfe peristaltischer Pumpen (Infusomat B. Braun Melsungen AG) superfundierte Hippokampus-Schnitt lag auf einem Stück Gaze. Eingeleitetes Carbogen hielt die erforderliche Sauerstoffzufuhr der Lösung aufrecht. Die Durchflussgeschwindigkeit betrug 200 ml/h.

Die Stimulation der CA2-Region (Schaffer-Kollateralen) erfolgte unter Verwendung eines Reizgenerators (Laborcomputer Labteam der Fa. MediSyst GmbH, Linden, DE) über eine Isoliereinheit mit Hilfe einer bipolar konzentrischen Stahlelektrode (Rhodes Medical Systems, USA. Die Impulsbreite betrug 200 µs, die Stromstärke konstant 200 µA. Der Reizgenerator löste im Abstand von jeweils 20 Sekunden 4 Einzelreizungen aus, die im Schnitt insgesamt 4 Populationsspikes evozierten. Die Ableitung der Antwort erfolgte in der CA3 Region. Das System mittelte die Reizantwort der 4 Spike-Amplituden.

Die Wirkung von Oenothera Extrakt auf das evozierte Potential wurde nach Einzelreizung wie auch nach Induktion der Langzeitpotenzierung durch einen kurzdauernden (1 s) tetanischen Reiz (90 Hz) untersucht (siehe K.G. Reymann, H.K. Matthies, U. Frey, V.S. Voborbyev, H. Matthies "Calcium-induced long-term potentiation in the hippocampal slice. Characterization of the time course and conditions"", Brain Bulletin (1986), 17: 291-296). Dabei wurde der Schnitt zunächst mit Kontrolllösung superfundiert. Nach Auffinden eines geeigneten Signals und Registrierung dieses Ausgangssignals während mehrerer Zeitpunkte wurde anstelle der Kontrolllösung auf Oenothera Extrakt-haltige Lösung umgeschaltet. Jeder Schnitt wurde jeweils nur zu einem Experiment benutzt. Werte werden für n=4 Schnitte angegeben.

Die Ableitung der evozierten Antwort erfolgte in 10-minütigem Abstand extrazellulär mit einer gezogenen Glaskapillare (Elektrodenpuller, Rhema Labortechnik, DE). Das Antwortsignal wurde verstärkt (Verstärker "LMI", List Electronics, Darmstadt, DE) mit einem Laborrechnersystem Labteam (Software NeuroTool, MediSyst GmbH, Linden DE) analog digital gewandelt (Auflösung 12 Bit) und ausgewertet. Nach Auffinden eines geeigneten Signals (Amplitudenhöhe etwa 1 mV) wurde die Amplitudenhöhe des Populationsspikes (SAP=Summenaktionspotential; Popspike) als Ausgangsgröße festgelegt. Dieser Referenzwert ergab sich aus dem Mittelwert der letzten drei gemessenen Amplitudenhöhen während Superfusion mit der ACSF Lösung (Ausgangswert). Danach erfolgte die Superfusion mit Testsubstanz mit gleichem Procedere. In Folgeexperimenten erfolgte eine kurzfristige tetanische Reizung (Theta-Burst-Stimulus=TBS) zur Induktion der Langzeitpotenzierung. Hieraus resultierende Amplitudenveränderungen wurden in % dieses Ausgangswertes (n=4) angegeben. Werte in Gegenwart der Testsubstanz sind in % Reduktion dieses Referenzwertes angegeben.

Die erste Versuchsanordnung zeigt eine Konzentrations-abhängige Abnahme der Amplitude des Populationsspikes in Gegenwart von 5, 10 und 20 mg/Liter Oenothera Extrakt bei Einzelreizung (siehe Abb. 5). Die Auslösung der Langzeitpotenzierung durch TBS wurde Konzentrations-abhängig verbessert (ebenfalls Abb. 5), wie es von Versuchen mit Medikamenten zur Behandlung von Demenzen wie Donepezil her bekannt ist.

Gleichartige Veränderungen wurden in der Vergangenheit auch in Gegenwart von Rasagilin, einem Medikament zur Behandlung der Parkinson'schen Krankheit sowie in Gegenwart von Paroxetin einem Medikament zur Behandlung von Depressionen beobachtet Abb. 5.

Die Untersuchung der Wirkung von Oenothera Extrakt im Hippokampus-Schnittpräparat zeigte eine Konzentrations-abhängige Abnahme des Populationsspikes der Pyramidenzellen. Bei Auslösung der Langzeit-Potenzierung (LTP) durch multiple elektrische Reizung wurde eine Abnahme der LTP gemessen, wie sie auch in der Vergangenheit in Gegenwart von Donepezil, einem Medikament, das bei Demenzen verschrieben wird, beschrieben wurde (Abb. 5). Eine Abnahme der LTP wird üblicherweise in Relation zu einer neuroprotektiven Wirkung gesehen (siehe W. Dimpfel, JA Hoffmann"Effects of rasagiline, ist metabolite aminoindan and selegiline in glutamate receptor mediated signalling in the rat hippocampus slice in vitro" BMC Pharmacology 2011;11:2). Alle Ergebnisse stehen mit der Vorstellung einer neuroprotektiven Wirkung im Einklang.

Die Versuchsergebnisse demonstrieren somit eine Wirkung von Oenothera Extrakt auf das glutamaterge System, da die Synapse zwischen Schaffer Kollateralen und Pyramidenzellen Glutamat als Transmitter benutzt.

Die vorgenannten Beispiele dienen der Erläuterung der Erfindung, ohne dass die Erfindung jedoch auf die speziell beschriebenen Ausführungsformen beschränkt sein soll.

## Patentansprüche

1. Oenothera Extrakt, Oenothera Extraktderivate und/oder dessen Inhaltsstoffe als Modulator des glutamatergen Systems.

2. Oenothera Extrakt, Oenothera Extraktderivate und/oder dessen Inhaltsstoffe nach Anspruch 1 als Modulator der Glutamatrezeptoren.

3. Oenothera Extrakt, Oenothera Extraktderivate und/oder dessen Inhaltsstoffe zur Behandlung von Zuständen, die durch eine Überaktivität des glutamatergen Systems bedingt sind.

4. Oenothera Extrakt, Oenothera Extraktderivate und/oder dessen Inhaltsstoffe nach Anspruch 3, wobei die Zustände zu einer Depression geführt haben.

5. Oenothera Extrakt, Oenothera Extraktderivate und/oder dessen Inhaltsstoffe zur Behandlung von Störungen des zentralen Nervensystems, insbesondere im Sinne einer **adaptogenen** Wirkung.

6. Oenothera Extrakt, Oenothera Extraktderivate und/oder dessen Inhaltsstoffe nach Anspruch 5, wobei die Störungen durch degenerative Erkrankungen, wie Demenzen, Morbus Parkinson und dem Erschöpfungs-Syndromm bedingt sind.

7. Oenothera Extrakt, Oenothera Extraktderivate und/oder dessen Inhaltsstoffe nach einem oder mehreren der Ansprüche 3 bis 6, **dadurch gekennzeichnet, daß** sie in einer Dosis von ca. 50 mg bis ca. 800 mg pro Tag, bevorzugt von ca. 200 mg - ca. 300 mg, besonders bevorzugt von ca. 250 mg pro Tag verabreicht, vorzugsweise oral verabreicht, werden.

8. Mittel enthaltend Oenothera Extrakt, Oenothera Extraktderivate und/oder dessen Inhaltsstoffe zur Behandlung von Zuständen, die durch eine Überaktivität des glutamatergen Systems bedingt sind.

9. Mittel nach Anspruch 8, wobei die Zustände durch eine Überaktivität der Glutamatrezeptoren, insbesondere auch der dopaminergen Rezeptoren bedingt sind.

10. Mittel enthaltend Oenothera Extrakt, Oenothera Extraktderivate und/oder dessen Inhaltsstoffe zur Behandlung von Störungen des zentralen Nervensystems, insbesondere bei degenerativen Erkrankungen, wie Demenzen, Morbus Parkinson und dem Erschöpfungssyndrom.

11. Mittel nach einem der Ansprüche 8 - 10, enthaltend einen therapeutisch wirksamen Gehalt an dem Wirkstoff und optional übliche Hilfs- und Zusatzstoffe.

12. Mittel nach einem der Ansprüche 8 - 11, **dadurch gekennzeichnet, daß** sie den Extrakt in einer Dosis von ca. 50 mg bis ca. 800 mg pro Tag, bevorzugt von ca. 200 mg - ca. 300 mg, besonders bevorzugt von ca. 250 mg pro Tag enthalten.

13. Mittel nach einem der Ansprüche 8 - 12, **dadurch gekennzeichnet, daß** sie zur oralen Verabreichung hergerichtet sind.

14. Mittel nach einem oder mehreren der Ansprüche 8 - 13 zur Behandlung von Depressionen und Stress-induzierten Erschöpfungszuständen (adaptogene Wirkung).

15. Mittel nach einem oder mehreren der Ansprüche 8 - 13 zur Behandlung von Burnout Syndromen im Sinne eines Nahrungsergänzungsmittels.
